# EUROPEAN PATENT SPECIFICATION

(11) **EP 4 493 671 B1**
(45) Date of publication and mention of the grant of the patent: **15.10.2025**
(21) Application number: 23723284.8
(22) Date of filing: 18.03.2023
(51) Int. Cl.: B33Y 30/00, B33Y 80/00, C12M 3/00, A61F 2/00, C12M 1/26, C12M 1/12

(54) **A REINFORCING AND SEALING CONSTRUCTION FOR A BIOPRINTED TISSUE MODEL**
VERSTÄRKUNGS- UND ABDICHTUNGSKONSTRUKTION FÜR EIN BIOGEDRUCKTES GEWEBEMODELL
CONSTRUCTION DE RENFORCEMENT ET D'ÉTANCHÉITÉ POUR UN MODÈLE DE TISSU BIO-IMPRIMÉ

(30) Priority: 18.03.2022 EP 22461526
(43) Date of publication of application: 22.01.2025
(73) Proprietor: Polbionica Sp. z o.o., 01-242 Warszawa (PL)
(72) Inventor: WSZOLA, Michal, 03-532 Warszawa (PL); KLAK, Marta, 64-920 Pila (PL); BERMAN, Andrzej, 00-565 Warszawa (PL); DOMANSKI, Sylwester, 03-821 Warszawa (PL); DOBRZANSKI, Tomasz, 02-987 Warszawa (PL); JANOWSKA, Oliwia, 02-503 Jastrzab (PL)
(74) Representative: AOMB Polska Sp. z.o.o.
(86) International application number: PCT/PL2023/050020
(87) International publication number: WO 2023/177316

(56) References cited:
- WO-A1-2015/160348
- WO-A1-2021/014359
- WO-A1-93/02635
- US-A1- 2022 025 309

## Description

The object of the invention is a reinforcing and sealing construction for a bioprinted tissue model.

The document WO1993002635A1 discloses a chamber made of a biocompatible material for implantation in the body, in which a material is contained which is immunologically compatible with the body, which comprises valves which ensure supporting the longevity of the contained biological material. The document does not describe the application of sealing bioink.

The document US5786216A describes a biocompatible capsule for containing cells for implantation, comprising an inner support, which provides the capsule with tensile strength. The document does not include information about the possibility of using bioink.

The document US20070276507A1 discloses a method for the reconstruction or replacement of laminarly organised organs or tissue structures, e.g. the bladder of a patient requiring such treatment, comprising the steps of providing a biocompatible synthetic or natural polymer matrix shaped to conform to at least a part of the luminal organ or tissue structure in need of said treatment; depositing a First cell population on or in a First surface of said polymer matrix; placing a second cell population in a second surface of said polymer matrix, and implanting the shaped polymer construct into the patient.

The document US11051509B2 discloses a device supporting the production and regeneration of tissues and organs, working by itself or in the human body. It describes the printing of living tissue inside a chamber with flows of fluids, cells, and growth factors, said chamber comprising at least one inflow and outflow port, which enables the circulation of fluids and other biological materials. Said chamber is made of biodegradable polymers, glass, plastics and/or metal, and it is configured in any shape which will accommodate the tissue.

The document CN111655835A describes hydrogels, comprising gelatin methacrylate, hyaluronic acid methacrylate, and optionally gelatin, collagen, fibrin/thrombin, Matrigel, agarose, hyaluron Antiramin, gelatin tyramine, and an alginate. On the other hand, the document does not include information about using a reinforcing casing for the organ being printed.

The document EP3146939B1 discloses an implantation tool comprising cells/a scaffold, and a surrounding device. The scaffold may be natural hydrogel comprising collagen, hyaluronic acid, an alginate, agarose, chitosan, fibrin, gelatin or copolymers thereof. The device may be used to repair cartilage.

The document US2022/025309A1 describes a device for culture of printed tissue comprising a base element forming a chamber adapted to receive the printed tissue. Before starting the printing operation, the whole surface of the base element is covered with bioink. The base is introduced into an external module provided with a cover and ports to allow flow of fluids.

Prior art describes numerous examples of applications of implantation devices, comprising cells, tissues or medicinal substances therein. Chambers can have perforated or semipermeable walls. However, in prior art there is no information about the application of sets for protecting/ensuring the integrity of an organ bioprinted *ex vivo,* which would comprise a casing and bioink allowing for sealing the casing.

The purpose of the invention was the development of a reinforcing and sealing construction for a bioprinted organ, whose main element is a casing with special sealing bioink. It ensures proper conditions during the steps of printing, cultivation in a bioreactor, as well as during implantation of an organ for a patient. It allows for maintaining sterility, proper deposition of blood vessels, and ensuring the tightness and strength of an organ after transplantation into a living organism.

The subject of the invention is a reinforcing and sealing construction for a bioprinted tissue model, which comprises a casing and sealing bioink, the casing comprising an inner module comprising a casing base and a perforated cover, and an outer module comprising a container comprising technological valves, an outer cover, and plugs for the technological valves.

Preferably, the casing base has a marker for calibrating printer heads.

Preferably, the casing is made of biocompatible materials, preferably resins with biocompatible properties and/or polymers with biocompatible properties.

Preferably, the volume of the casing is adjusted to the volume of the bioprinted tissue model.

Preferably, a space with a width of no less than 1.0 mm is provided between the bioprinted tissue model and the inner module, and a space with a width of no less than 0.5 mm is provided between the inner module and the outer module.

Preferably, the bioink comprises in its composition a buffer solution of an extracellular matrix.

Preferably, a buffer solution comprising an extracellular matrix, methacrylated hyaluronic acid as well as methacrylated gelatin and a photoinitiator, preferably lithium phenyl-2,4,6-trimethylbenzoylphosphinate, is used as the sealing bioink.

Preferably, a buffer solution comprising one part of an extracellular matrix with a concentration ranging from 1 to 10% (w/v) and one part of a mixture of methacrylated hyaluronic acid with a concentration ranging from 1 to 3% (w/v) and methacrylated gelatin with a concentration ranging from 5 to 25% (w/v) as well as an addition of LAP photoinitiator in an amount not exceeding a final concentration of 0.5% (w/v) is used as the sealing bioink, the ratio of methacrylated hyaluronic acid to methacrylated gelatin in the mixture being 1 : 0.67.

The method for assembling the reinforcing and sealing construction comprises the steps:
a) Filling the space between the bioprinted tissue model and the casing base with sealing bioink;
b) covering the casing base with the bioprinted tissue model by the perforated cover, and pressing it in order to create an inner module;
c) optionally, refilling the space between the bioprinted tissue model and the casing base chamber with sealing bioink;
d) sliding the folded inner module into the container in order to create an outer module;
e) closing the outer module by means of the cover;
f) optionally, adding sealing bioink through a technological valve;
g) installing plugs in the technological valves.

The object of the invention is presented in embodiments in the drawing, where:
Fig. 1 presents a perspective view of the reinforcing casing according to the invention
Fig. 2 presents the disassembled elements of the casing

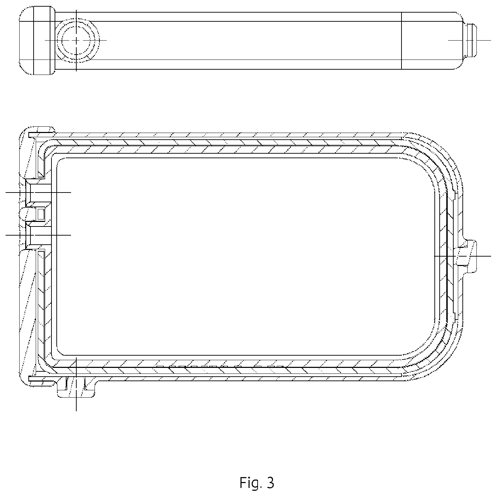

Fig. 3 presents a top and side view of an assembled casing
Fig. 4 presents a table summarising stability tests and an assessment of the usefulness of selected formations of sealing bioinks. The Following formulations have been tested: a: 10% dECM hydrogel + GelMa + HaMa, b: 10% dECM hydrogel (+ GelMa + HaMa) + 1.5% agarose 1:1, c: dECM5% + 1.5% agarose (1:1), d: 5% dECM hydrogel (+ GelMa + HaMa) + 1.5% agarose 2:1, e: 5% dECM hydrogel (+ GelMa + HaMa) + 3% agarose 2:1, f: 1% chitosan-based hydrogel + 2% agarose, g: dECM 5% + GelMa + HaMa.

The advantage of the invention is the fact that the entirety of the invention consists of a number of designed and manufactured elements. They ensure proper performance of the bioprinting process for the tissue model. Among these elements, one can list: the casing of the bioprinted model, and sealing bioink. The casing of the model in turn consists of two modules: an inner one, and an outer one.

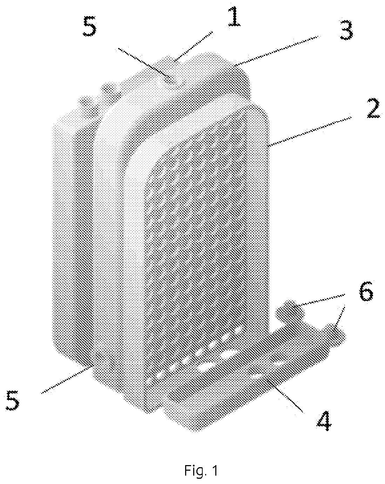

Fig. 1 presents a perspective view of the casing. The casing is assembled From elements in the consecutive steps of the bioprinting process, so that a properly functioning tissue model can be produced in the end. The casing base 1 of the inner module is placed in a bioprinter, becoming filled in a special and planned manner with bioink, with or without a biological material.

Such construction of the casing ensures a number of functions of the used construction. The inner module of the casing serves a supporting and isolating function, the casing base 1 enabling the transport of the printed tissue model. Moreover, the casing base 1 can have a marker 7, to which printer heads are calibrated, thus serving a calibrating function. The casing has valves for the deposition of vessels, which ensure their proper position and orientation with respect to the printed tissue model. Apart From this, the casing base 1 of the casing has valves which provide support and allow for immobilising the vessels relative to the tissue model, serving a supporting Function for the vessels. Besides that, the inner module has been designed and manufactured so as to ensure the ability to introduce sealing bioink between the printed tissue model and the casing. A space has been left between the side walls of the casing base 1 and the printed tissue model. The perforated cover 2 allows for the introduction of sealing bioink so as to surround the entire tissue model, reaching every free space.

The outer module in turn serves functions related to ensuring mechanical strength, and ensuring the tightness of the entire tissue model; it has a supporting function, since the casing provides the possibility of installing the tissue model in a bioreactor chamber. In addition, a properly selected shape and placement of the inlet and outlet vessels provide the possibility of implanting the tissue model into the recipient's body. The outer module consists of a container 3 comprising at least two technological valves 5, an outer cover 4, and plugs 6.

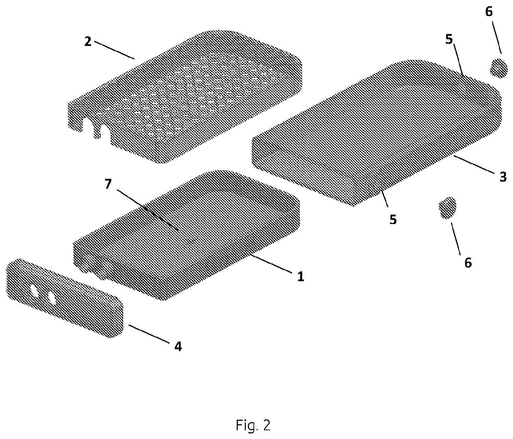

Fig. 2 presents the elements of a disassembled casing. The method of assembling the casing and sealing it by means of sealing ink involves filling the space between the bioprinted organ and the casing base 1 with sealing bioink. Subsequently, the casing base 1 with the bioprinted tissue model are covered by the perforated cover 2, and it is pressed in order to create an inner module. The perforated cover 2 may be optionally used in order to resupply the sealing bioink. Subsequently, the inner module is slid into the container 3 in order to create an outer module. The outer module is closed by means of the cover 4. The resulting construction is arranged in a vertical position, with the outlet of the vessels facing upwards. Subsequently, sealing bioink is injected through the lower technological valve 5. Sealing bioink is fed until the sealing bioink is observed in the upper technological valve 5. It is necessary to check the correctness of filling; that is, visually assess the presence of air bubbles. If necessary, the casing is tilted with one of its technological valves 5 upwards, and the sealing bioink is resupplied. Finally, the plugs 6 are installed in the technological valves and pressed, so that the rim of a plug 6 would rest against the wall of the casing.

The function of ensuring tightness is one of the more important parameters, and it is the result of the sum of the applied solutions: the casing closure method, the method of supporting and depositing vessels, the type and usage method of sealing bioink.

The casing is made of biocompatible materials. All of its elements are made of biocompatible silicone, biocompatible resins or biocompatible polymers. Apart from fulfilling the condition of biocompatibility and having proper mechanical properties, the material must ensure the possibility of sterilisation in high temperature and/or radiation sterilisation.

Biocompatible materials are characterised by proper actions in a living organism. A material with high biocompatibility should be characterised by the Following Features: no toxicity; no impact on the immunity system of the body; not causing haemolysis.

The casing may be prepared in various sizes; the most important thing is to retain its individual components and formula. The size of the casing must be designed so as to provide the possibility of bioprinting a tissue model in the requested volume, and so as to provide the possibility of application of sealing bioink.

The sealing bioink in turn serves functions related to providing proper biological conditions for the tissue model being printed; it fills the spaces between elements of the casing and the tissue model, ensuring adhesion of the main tissue model and elements of the casing to bioink, and improves the mechanical properties of the bioprinted tissue model.

One of the components of the sealing bioink is an extracellular matrix produced according to the patent application WO2021014359A1. Changes introduced to the procedure, like the used antibiotic or small conveniences, e.g. during pouring, have no real impact on the entirety of the performed process.

Methacrylated components (e.g. methacrylated gelatin or methacrylated hyaluronic acid) are the carrier; glycerol is the lubricant. LAP (lithium phenyl-2,4,6-trimethylbenzoylphosphinate) is the photoinitiator.

A number of varieties of sealing bioink were tested in the performed studies:
- A 1.5% agarose solution;
- Chitosan-based hydrogel with an addition of beta-glycerophosphate (beta-GP);
- 5% dECM hydrogel + GelMa + HaMa;
- 10% dECM hydrogel + GelMa + HaMa;
- 15% dECM hydrogel + GelMa + HaMa;
- 5% dECM hydrogel + dECM powder + GelMa + HaMa;
- 10% dECM hydrogel + ECM powder + GelMa + HaMa;
- 20% GelMa
- 10% GelMa
- dECM hydrogel (+ GelMa + HaMa) + agarose:
- 10% dECM hydrogel (+ GelMa + HaMa) + 1.5% agarose (1:1);
- 5% dECM hydrogel (+ GelMa + HaMa) + 1.5% agarose (1:1);
- 5% dECM hydrogel (+ GelMa + HaMa) + 1.5% agarose (2:1);
- 5% dECM hydrogel (+ GelMa + HaMa) + 3% agarose (2:1);
- Chitosan-based hydrogel + 1.5% agarose (1:1);
- Chitosan-based hydrogel with an addition of beta-glycerophosphate (beta-GP) + 1.5% agarose (1:1);
- 1.5% agarose + cellulose (1:1 and 4:1).

The invention is presented in non-limiting embodiments:

### Embodiment 1.

### Casing production technology

Strength tests involving the measurement of pressure limit for a bioprinted tissue model constitute a basis for the necessity to use sealing bioink and each element of the model. The conditions of the pressure limit which must be met by the model depend on the physiological pressure, specific to the species and the research model. Pressure tests were performed in order to prove the validity of using sealing bioink. The tests were performed on models without sealing bioink, as well as those which used it.

After the completion of printing, preparation of the model for pressure supply strength tests was commenced. Firstly, the vascular system of the tissue model was unclogged by removing supporting bioink. Subsequently, specially designed adapters were mounted to the pancreas, which were supposed to enable the connection of drains, and as a consequence the flow of the medium. The experiment was performed for 2 types of the model: with and without the use of sealing bioink.

In order to ensure continuous flow of fluid (optimally, 50-100 ml/min.), the strength tests began with the stabilisation of flow across the pancreas, and the elimination of any air bubbles as well as the residual bioink content of the entire system. Subsequently, a pressure increase was commenced in the system. The tests were performed until the time of visible leakage of the model.

Models without sealing bioink exhibited first leakage near channel entrances, at a pressure value of 15-29 mmHg. On the other hand, models which used sealing with the developed bioink exhibited first leakage at a value of 32-49 mmHg. The test involved a total of 8 Full-size models. Therefore, it indicated fully justified application of sealing bioink.

It was a test of the properties of sealing bioink-it did not use a full reinforcing casing. The use of bioink and a casing allows for maintaining the pressure at a level as high as 400 mmHg.

The filling of empty spaces between the bionic tissue model and the reinforcing casing with dECM-based hydrogel allowed for producing a satisfactory composition of the sealing biocoating, also exhibiting its full adhesion to the bioink used to print the tissue model. Such bioink filled all the gaps between the bionic organ and the reinforcing layer, providing protection against delamination, and therefore leakage of the bionic tissue model.

### Embodiment 2.

### The composition of sealing bioink

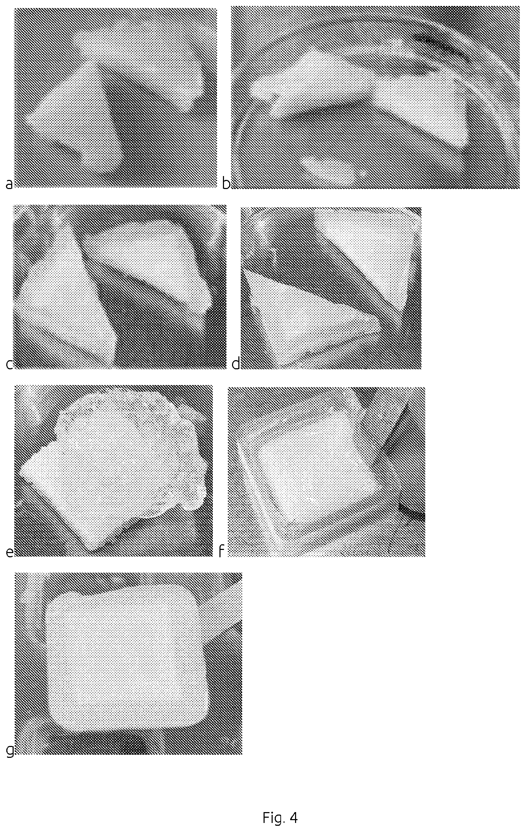

A number of bioink varieties with different compositions have been tested. Sample results of the tests performed for sealing bioinks with various compositions are presented in Fig. 4. The decellularization material was acquired from a local slaughterhouse, and directly after preparation, the pancreas tissues were precisely cleaned of fat, large vessels and connective tissue, and stored in a PBS solution prior to Further handling. The entire process was conducted in accordance with the protocol published by: Klak M, Int J Mol Sci 2021;22:1-16. https://doi.org/10.3390/ijms22137005

**Table 1. The tested varieties of sealing bioink**

| Hydrogel composition |
|---|
| 5% dECM hydrogel + GelMa + HaMa |
| 10% dECM hydrogel + GelMa + HaMa |
| 10% dECM hydrogel (+ GelMa + HaMa) + 1.5% agarose 1:1 |
| 5% dECM + 1.5% agarose (1:1) |
| 5% dECM hydrogel (+ GelMa + HaMa) + 1.5% agarose 2:1 |
| 5% dECM hydrogel (+ GelMa + HaMa) + 3% agarose 2:1 |
| 1% chitosan-based hydrogel + 2% agarose |
| - Chitosan-based hydrogel with an addition of beta-glycerophosphate (beta-GP) + 1.5% agarose (1:1); |

The sealing bioink with the most preferable Features was selected From the performed tests: 5% dECM hydrogel + GelMa + HaMa.

The selected sealing bioink is characterised by:
- high stability within the range of physiological temperatures,
- does not exhibit cytotoxicity,
- stably adheres to bioink forming the mass of the bionic model,
- undergoes crosslinking due to light with a wavelength of 405 nm,
- exhibits low viscosity,
- retains Fluidity under the conditions of the experiment, allowing for sufficient penetration of the model,
- exhibits a high DS for methacrylates, in order to provide stable Fillings,
- high flexibility of material after crosslinking, not causing delaminations.

### Embodiment 3:

### The crosslinking procedure:

Multistep crosslinking was used, with an extended time of exposure to light with a wavelength of 405 nm or 365 nm. Firstly, layers were crosslinked between the bionic pancreas and the inner layer of the reinforcing casing, followed by crosslinking bioink fed between an previously crosslinked reinforcing layer and the outer part of the casing-in this case, sealing bioink was introduced via technological valves in the outer casing. Crosslinking parameters:
- a wavelength of 405 nm or 365 nm;
- duration of crosslinking from 60 s to 2160 s (optimally 1440 s);
- power from 1 to 30 mW/cm² (optimally 28.5 mW/cm²)-for a light wavelength of 405 nm, and From 1 to 15 mW/cm² for a light wavelength of 365 nm

## Claims

1. A **reinforcing and sealing construction** for a bioprinted tissue model, comprising a casing and sealing bioink, the casing comprising an inner module comprising a casing base (1) and a cover (2), and an outer module comprising a container (3) comprising technological valves (5), an outer cover (4) and plugs (6) for technological valves (5) **characterised in that** a cover is perforated.

2. The reinforcing and sealing construction according to claim 1, **characterised in that** the casing base (1) has a marker for calibrating (7) printer heads.

3. The reinforcing and sealing construction according to claim 1 or 2, **characterised in that** the casing is made of biocompatible materials, preferably resins with biocompatible properties and/or polymers with biocompatible properties.

4. The reinforcing and sealing construction according to any of the claims 1-3, **characterised in that** the volume of the casing is adjusted to the volume of the bioprinted tissue model.

5. The reinforcing and sealing construction according to any of the claims 1-4, **characterised in that** a space with a width of no less than 1.0 mm is provided between the bioprinted tissue model and the inner module, and a space with a width of no less than 0.5 mm is provided between the inner module and the outer module.

6. The reinforcing and sealing construction according to any of the claims 1-5, **characterised in that** the bioink comprises in its composition a buffer solution of an extracellular matrix.

7. The reinforcing and sealing construction according to any of the claims 1-6, **characterised in that** a buffer solution comprising an extracellular matrix, methacrylated hyaluronic acid as well as methacrylated gelatin and a photoinitiator, preferably lithium phenyl-2,4,6-trimethylbenzoylphosphinate, is used as sealing bioink.

8. The reinforcing and sealing construction according to any of the claims 1-7, **characterised in that** a buffer solution comprising one part of an extracellular matrix with a concentration ranging from 1 to 10% (w/v) and one part of a mixture of methacrylated hyaluronic acid with a concentration ranging from 1 to 3% (w/v) and methacrylated gelatin with a concentration ranging from 5 to 25% (w/v) as well as an addition of LAP photoinitiator in an amount not exceeding a final concentration of 0.5% (w/v) is used as sealing bioink, the ratio of methacrylated hyaluronic acid to methacrylated gelatin in the mixture being 1 : 0.67.

## Patentansprüche

1. Eine Verstärkungs- und Abdichtungskonstruktion für ein biogedrucktes Gewebemodell, umfassend eine Hülle und ein abdichtendes Bioink, wobei die Hülle ein Innenmodul mit einer Gehäusebasis (1) und einer Abdeckung (2) sowie ein Außenmodul mit einem Behälter (3), der technologische Ventile (5) umfasst, einer Außenabdeckung (4) und Verschlüssen (6) für die technologischen Ventile (5) aufweist, **dadurch gekennzeichnet, dass** die Abdeckung perforiert ist.

2. Die Verstärkungs- und Abdichtungskonstruktion nach Anspruch 1, **dadurch gekennzeichnet, dass** die Gehäusebasis (1) eine Markierung (7) zur Kalibrierung von Druckköpfen aufweist.

3. Die Verstärkungs- und Abdichtungskonstruktion nach Anspruch 1 oder 2, **dadurch gekennzeichnet, dass** das Gehäuse aus biokompatiblen Materialien hergestellt ist, vorzugsweise aus Harzen mit biokompatiblen Eigenschaften und/oder Polymeren mit biokompatiblen Eigenschaften.

4. Die Verstärkungs- und Abdichtungskonstruktion nach einem der Ansprüche 1-3, **dadurch gekennzeichnet, dass** das Volumen des Gehäuses an das Volumen des biogedruckten Gewebemodells angepasst ist.

5. Die Verstärkungs- und Abdichtungskonstruktion nach einem der Ansprüche 1-4, **dadurch gekennzeichnet, dass** zwischen dem biogedruckten Gewebemodell und dem Innenmodul ein Abstand mit einer Breite von mindestens 1,0 mm vorgesehen ist und zwischen dem Innenmodul und dem Außenmodul ein Abstand mit einer Breite von mindestens 0,5 mm vorgesehen ist.

6. Die Verstärkungs- und Abdichtungskonstruktion nach einem der Ansprüche 1-5, **dadurch gekennzeichnet, dass** das Bioink in seiner Zusammensetzung eine Puffermischung einer extrazellulären Matrix umfasst.

7. Die Verstärkungs- und Abdichtungskonstruktion nach einem der Ansprüche 1-6, **dadurch gekennzeichnet, dass** als abdichtendes Bioink eine Pufferlösung verwendet wird, die eine extrazelluläre Matrix, methakryliertes Hyaluronsäure sowie methakryliertes Gelatine und einen Photoinitiator, vorzugsweise Lithiumphenyl-2,4,6-trimethylbenzoylphosphinat, umfasst.

8. Die Verstärkungs- und Abdichtungskonstruktion nach einem der Ansprüche 1-7, **dadurch gekennzeichnet, dass** als abdichtendes Bioink eine Pufferlösung verwendet wird, die einen Anteil einer extrazellulären Matrix mit einer Konzentration von 1 bis 10 % (w/v) sowie einen Anteil eines Gemisches aus methakrylierter Hyaluronsäure mit einer Konzentration von 1 bis 3 % (w/v) und methakrylierter Gelatine mit einer Konzentration von 5 bis 25 % (w/v) enthält, wobei zusätzlich ein LAP-Photoinitiator in einer Menge zugesetzt ist, die eine Endkonzentration von 0,5 % (w/v) nicht überschreitet, und wobei das Verhältnis von methakrylierter Hyaluronsäure zu methakrylierter Gelatine in dem Gemisch 1 : 0,67 beträgt.

## Revendications

1. Construction de renforcement et d'étanchéité pour un modèle de tissu bioconstruit, comprenant une enveloppe et une bio-encre d'étanchéité, ladite enveloppe comprenant un module interne comprenant une base de boîtier (1) et un couvercle (2), ainsi qu'un module externe comprenant un récipient (3) comprenant des valves technologiques (5), un couvercle externe (4) et des bouchons (6) pour lesdites valves technologiques (5), **caractérisée en ce que** le couvercle est perforé.

2. Construction de renforcement et d'étanchéité selon la revendication 1, **caractérisée en ce que** la base de boîtier (1) comporte un repère (7) pour le calibrage des têtes d'impression.

3. Construction de renforcement et d'étanchéité selon la revendication 1 ou 2, **caractérisée en ce que** l'enveloppe est réalisée en matériaux biocompatibles, de préférence des résines présentant des propriétés biocompatibles et/ou des polymères présentant des propriétés biocompatibles.

4. Construction de renforcement et d'étanchéité selon l'une quelconque des revendications 1 à 3, **caractérisée en ce que** le volume de l'enveloppe est adapté au volume du modèle de tissu bioconstruit.

5. Construction de renforcement et d'étanchéité selon l'une quelconque des revendications 1 à 4, **caractérisée en ce que** un espace d'une largeur d'au moins 1,0 mm est prévu entre le modèle de tissu bioconstruit et le module interne, et un espace d'une largeur d'au moins 0,5 mm est prévu entre le module interne et le module externe.

6. Construction de renforcement et d'étanchéité selon l'une quelconque des revendications 1 à 5, **caractérisée en ce que** la bio-encre comprend dans sa composition une solution tampon d'une matrice extracellulaire.

7. Construction de renforcement et d'étanchéité selon l'une quelconque des revendications 1 à 6, **caractérisée en ce que** la solution tampon utilisée comme bio-encre d'étanchéité comprend une matrice extracellulaire, de l'acide hyaluronique méthacrylé, ainsi que de la gélatine méthacrylée et un photo-initiateur, de préférence le lithium phényl-2,4,6-triméthylbenzoylphosphinate.

8. Construction de renforcement et d'étanchéité selon l'une quelconque des revendications 1 à 7, **caractérisée en ce que** la bio-encre d'étanchéité est constituée d'une solution tampon comprenant une partie de matrice extracellulaire avec une concentration comprise entre 1 et 10 % (p/v), et une partie d'un mélange d'acide hyaluronique méthacrylé avec une concentration comprise entre 1 et 3 % (p/v) et de gélatine méthacrylée avec une concentration comprise entre 5 et 25 % (p/v), ainsi qu'un ajout de photo-initiateur LAP en une quantité n'excédant pas une concentration finale de 0,5 % (p/v), le rapport entre l'acide hyaluronique méthacrylé et la gélatine méthacrylée dans ledit mélange étant de 1 : 0,67.
